# EUROPEAN PATENT APPLICATION

(11) **EP 2 011 840 A1**
(43) Date of publication of application: **07.01.2009**
(21) Application number: 07741828.3
(22) Date of filing: 18.04.2007
(51) Int. Cl.: C09D 11/00, G01N 21/78, G01N 31/00

(54) **INK COMPOSITION FOR OZONE DETECTION AND OZONE INDICATOR**

(30) Priority: 20.04.2006 JP 2006117015
(71) Applicant: SAKURA COLOR PRODUCTS CORPORATION, Osaka-shi, Osaka 537-0025 (JP)
(72) Inventor: KAKIMOTO, Chizuko, Shijonawate-shi, Osaka 575-0033 (JP)
(74) Representative: Manley, Nicholas Michael
(86) International application number: PCT/JP2007/058392
(87) International publication number: WO 2007/123139

(57) **Abstract**

The present invention relates to an ink composition for ozone detection, and an ozone indicator using the composition. In particular, the present invention is mainly directed to provide an indicator capable of detecting ozone at high accuracy, even in a high-concentration ozone gas atmosphere, or in a high-concentration ozone gas atmosphere containing water vapor.

The ink composition for ozone detection of the present invention comprises an oxazine dye and a cationic surfactant, and does not comprise colorants (other than the oxazine dyes) that undergo a color change upon exposure to ozone.

## Description

### TECHNICAL FIELD

The present invention pertains to an ink composition for ozone detection and an ozone indicator using the composition.

### BACKGROUND ART

Because of its strong germicidal action and other abilities, ozone has been utilized for the sterilization and disinfection of foods, utensils, etc., as well as for the sterilization, disinfection and deodorization of confined atmospheres, such as hospital operating rooms.

Currently, ozone is used for the sterilization and disinfection of medical devices such as endoscopes, gastrocameras and the like. Accordingly, a variety of ozone disinfectors (sterilization apparatuses) have been developed. Sterilization methods that utilize the disinfectors, such as methods involving the use of ozone gas in a vapor phase, the immersion of medical devices in ozone water, etc. have been suggested. During such sterilizations, an indicator is required to verify whether the treated medical device object received the prescribed ozone sterilization. Known indicators, used as ozone detectors, have a color-change layer on a substrate thereof (Patent Document 1, etc.).
Patent Document 1: Japanese Unexamined Patent Publication No. H11-140360

### DISCLOSURE OF THE INVENTION

### Problems to be Solved by the Invention

In the ozone treatment of medical devices etc., ozone gas is used at concentration levels as high as hundreds of ppm. However, in conventional ozone indicators, color-change layers thereof undergo a color change in low-concentration ozone, making it difficult to verify whether the treated objects were actually exposed to high-concentration ozone.

Further, it is difficult for these indicators to detect ozone at high accuracy in atmospheres containing relatively large amounts of water vapor. For example, in the sterilization using ozone water (treated water), ozone gas generated from the ozone water is detected to verify whether sterilization was actually performed. However, since such ozone gas contains a large amount of water vapor, there may be cases where the conventional ozone indicators fail to detect ozone with desired accuracy.

In view of the above, a main object of the present invention is to provide an indicator capable of detecting ozone at higher accuracy, particularly in high-concentration ozone gas atmospheres, even high-concentration ozone gas atmospheres containing water vapor.

### Means for Solving the Problems

In view of the problems of the prior art, the present inventors conducted extensive research and found that the above object can be achieved by an ink composition containing a specific ingredient. The present invention has been accomplished based on such findings.

Specifically, the present invention relates to the following ink composition for ozone detection and an ozone indicator.
Item 1. An ink composition for ozone detection comprising an oxazine dye and a cationic surfactant, and not comprising colorants (other than the oxazine dyes) that undergo a color change upon exposure to ozone.
Item 2. The ink composition for ozone detection according to Item 1, wherein the cationic surfactant is a quaternary ammonium salt-type cationic surfactant.
Item 3. The ink composition for ozone detection according to Item 2, wherein the cationic surfactant is an alkyl trimethylammonium salt.
Item 4. The ink composition for ozone detection according to Item 1, further comprising an extender.
Item 5. The ink composition for ozone detection according to Item 1, further comprising a resinous binder.
Item 6. An ozone indicator comprising a first-color change layer formed from the ink composition for ozone detection according to Item 1.
Item 7. The ozone indicator according to Item 6, comprising, on a substrate, (1) at least one layer selected from (a) a second-color change layer (not including oxazine dyes) that undergoes a color change upon exposure to ozone, and (b) a non-color change layer that does not undergo a color change upon exposure to ozone; and (2) the first-color change layer, wherein each of the layers are laminated in such a manner that a part or all of each color-change layer can be exposed to an ozone atmosphere.
Item 8. The ozone indicator according to Item 7, having at least one second-color change layer.
Item 9. The ozone indicator according to Item 8, wherein the first-color change layer and second-color change layer undergo a color change upon different ozone concentrations and/or ozone exposure times.
Item 10. The ozone indicator according to Item 6 for use in detecting ozone in an ozone-gas atmosphere with a CT value of 1000 ppm-min or more.
Item 11. The ozone indicator according to Item 10, wherein atmospheric humidity is 90% or more.

### EFFECT OF THE INVENTION

The ink composition for ozone detection according to the present invention comprises, as essential components, an oxazine dye and a cationic surfactant, making it possible to provide an ozone indicator exhibiting different properties from those of known indicators.

The ozone indicator according to the present invention must detect a particularly higher concentration of ozone gas before it undergoes a color change. In addition, the ozone indicator is capable of accurately detecting ozone even in atmospheres containing water vapor. For the above reasons, the ozone indicator of the present invention is preferably utilized for the sterilization of, using high-concentration ozone, medical devices and the like. In particular, the ozone indicator of the invention is most suitably used in an apparatus using ozone water for sterilization, to detect ozone gas generated from the ozone water.

### BEST MODE FOR CARRYING OUT THE INVENTION

### 1. Ink Composition for Ozone Detection

The ink composition for ozone detection according to the present invention comprises an oxazine dye and a cationic surfactant, and does not comprise colorants (other than the oxazine dyes) that undergo a color change upon exposure to ozone.

Usable oxazine dyes are not critical, as long as the dye contains at least one oxazine ring having Formulas (I) to (III) below. Examples include monooxazine dyes that have one oxazine ring, and dioxazine dyes that have two oxazine rings, etc.

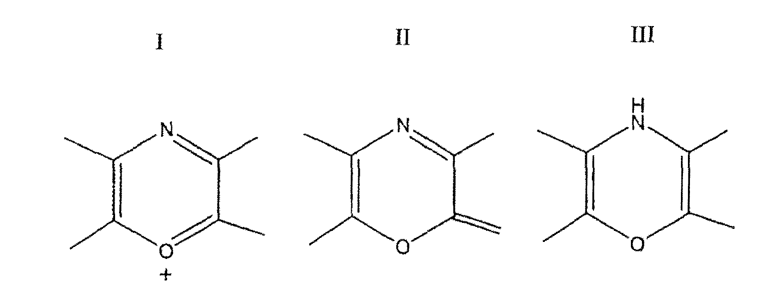

In the present invention, as an auxochrome, at least one of basic dyes comprising at least one substituted or unsubstituted amino group, and chrome mordant dyes comprising, as a substituent, an OH group, a COOH group, etc., may be employed.

Such oxazine dyes can be used singly or in a combination of two or more. Usable oxazine dyes may be known or commercially available products. Preferable examples of such oxazine dyes include, by dye number, C. I. Basic Blue 3, C. I. Basic Blue 12, C. I. Basic Blue 6, C. I. Basic Blue 10, C. I. Basic Blue 96, and the like. Among these, C. I. Basic Blue 3 etc. are more preferred.

The content of the oxazine dye varies depending on the type to be used, the desired color-changing properties (detection accuracy), etc. However, it is preferable that the proportion of the oxazine dye usually be from 0.01 wt.% to 10 wt.%, in particular 2 wt.% to 4 wt.%, relative to the composition of the present invention. By setting the proportion within the range specified above, ozone-detection sensitivity can be obtained more stably even in atmospheres containing water vapor.

As the cationic surfactant, a quaternary ammonium salt type-cationic surfactant may preferably be used. Usable quaternary ammonium salt-type cationic surfactants (hereunder sometimes simply referred to as "cationic surfactants") are not limited. Usually employed are alkyl ammonium salts, which may be a commercially available product. These can be used singly or in a combination of two or more. In the present invention, the use of these cationic surfactants makes it possible to achieve the more excellent color-change effect.

Preferred among these cationic surfactants are alkyl trimethylammonium salts, dialkyl dimethyl ammonium salts, and the like. Specific examples include coconut alkyl trimethyl ammonium chloride, tallow alkyl trimethyl ammonium chloride, behenyltrimethylammonium chloride, dodecyltrimethylammonium chloride, hexadecyltrimethylammonium chloride, lauryltrimethylammonium chloride, octadecyltrimethylammonium chloride, dioctyldimethylammonium chloride, distearyldimethylammonium chloride, alkylbenzyldimethylammonium chloride, and the like. Particularly preferred is lauryltrimethylammonium chloride.

The content of the cationic surfactant varies depending on the types of cationic surfactants employed, the desired color-changing property (detection accuracy), etc. However, it is preferable that the proportion of the cationic surfactants usually be from 0.01 wt.% to 5 wt%, in particular 0.1 wt.% to 2 wt.%, relative to the composition of the present invention. By setting the proportion within the range specified above, ozone-detection sensitivity can be more effectively controlled.

In addition to the above, other components used in known ink compositions, such as colorants other than oxazine dyes, resinous binders, extenders, solvents, etc., may be suitably added, as necessary, to the ink composition of the present invention.

The resinous binders usable herein may be suitably selected depending on the types of the substrate, etc. For example, known resin components included in writing, printing or other ink compositions are applicable as they are. Specific examples include maleic acid resin, amide resin, ketone resin, alkylphenol resin, rosin modified resin, polyvinyl butyral, polyvinyl pyrrolidone, cellulose resin, and the like. These can be used singly or in a combination of two or more.

The content of such a resinous binder is preferably 1 wt.% to 25 wt.%, in particular 1 wt.% to 20 wt.%, relative to the composition of the present invention. By setting the proportion within the range specified above, a more excellent coating film (printing layer) can be formed.

Examples of usable extenders include, but are not limited to, bentonite, activated clay, aluminium oxide, silica gel, and the like. In addition to the above, substances known as extender pigments may be employed. These can be used singly or in a combination of two or more. Preferred among these are porous extenders; in particular, silica gel is more preferred. The addition of such an extender mainly allows for the control of detection sensitivity.

It is preferable that the content of such an extender usually be from 1 wt.% to 20 wt.%, and in particular 1 wt.% to 10 wt.% relative to the composition of the present invention. By setting the proportion within the range specified above, a smoother coating film surface (printing surface) can be obtained.

The solvents usable in the present invention may be any of those usually used in printing, writing, or other ink compositions. Examples include solvents such as alcohol solvents, ester solvents, ether solvents, ketone solvents, hydrocarbon solvents, and the like. The solvents may be suitably selected according to the solubility etc. of a dye and a resinous binder to be employed. These can be used singly or in a combination of two or more.

Usable colorants other than oxazine dyes include any colorants that do not undergo a color change upon exposure to ozone (non-color change pigment). Usable non-color change pigments may be any known pigments, such as a normal color ink, etc.

It is preferable that the content of such a non-color change pigment usually be from 0.1 wt.% to 5 wt.%, in particular 1 wt.% to 4 wt.%, relative to the composition of the present invention.

The method for preparing the ink composition of the present invention is not critical. The aforesaid components may be combined together at one time, or sequentially to be uniformly mixed with a known agitator such as homogenizer, dissolver, etc. For example, an oxazine dye, a cationic surfactant, a resin binder, an extender, etc. may first be added sequentially to a solvent to be mixed and stirred.

The ink composition of the present invention may be used by itself. Alternatively, the ink composition may be, where necessary, carried on a substrate by allowing the ink composition to be applied, impregnated, etc. to the substrate. Examples of usable substrates include, but are not limited to, any suitable materials, such as paper sheets, wood materials, plastics, metals and alloys, ceramics, and cement and concretes, or like composite materials of these. Such materials may be suitably selected according to where and how the ink composition will be used, etc.

### 2. Ozone Indicator

The present invention also encompasses the ozone indicator comprising a first-color change layer formed from the aforesaid ink composition for ozone detection.

The indicator according to the present invention may be formed only from the first-color change layer, or may further include other layers, as necessary. For example, the present invention encompasses an ozone indicator comprising, on a substrate thereof, (1) at least one layer selected from (a) a second-color change layer (not including oxazine dyes) that undergoes a color change upon exposure to ozone, and (b) a non-color change layer that does not undergo a color change upon exposure to ozone; and (2) the first-color change layer. Such layers are laminated so that a part or all of each color-change layer is exposed to the ozone atmosphere.

The usable substrates in the ozone indicator of the present invention are not critical as long as such a substrate can include a color-change layer, or a non-color change layer formed thereon. The same types of substrates mentioned earlier may be employed.

The indicator according to the present invention may comprise, in addition to the first-color change layer, at least one layer selected from the aforesaid (a) non-color change layer, and (b) second-color change layer.

Such a non-color change layer can be formed from an ink composition containing a non-color change pigment that does not undergo a color change upon exposure to ozone. Such an ink composition usable herein may be a commercially available normal color ink. Examples include aqueous inks, oil-based inks, solventless inks, etc. For printing, known relief printing inks, gravure inks, screen printing inks, offset inks, etc. can be suitably and properly selected according to the printing method. These inks may be used singly as is, or mixed in a combination of two or more for toning. The ink contained in the non-color change layer may comprise substances (e.g., resinous binders, extenders, solvents, etc.) that are included in known ink compositions.

The second-color change layer can be formed from an ink composition containing a colorant other than oxazine dyes; and the colorant undergoes a color change upon exposure to ozone. Other than the colorants, the same compositions as those contained in the ink composition of the present invention are applicable.

It is preferable to use, as the colorant used for the second-color change layer, at least one dye particularly selected from anthraquinone dyes, azo dyes, and methine dyes.

The anthraquinone dye usable herein is not limited as long as it has an anthraquinone nucleus. Known anthraquinone disperse dyes can also be used. In particular, an anthraquinone dye having an amino group is preferred. It is still more preferable that the anthraquinone dye has at least one amino group selected from primary and secondary amino groups. In such a case, the dye may have two or more amino groups of the same or different kinds.

Specific examples include 1, 4-diaminoanthraquinone (C. I. Disperse Violet 1), 1-amino-4-hydroxy-2-methylaminoanthraquinone (C. I. Disperse Red 4), 1-amino-4-methylaminoanthraquinone (C. I. Disperse Violet 4), 1, 4-diamino-2-methoxyanthraquinone (C. I. Disperse Red 11), 1-amino-2-methylanthraquinone (C. I. Disperse Orange 11), 1-amino-4-hydroxyanthraquinone (C. I. Disperse Red 15), 1, 4, 5, 8-tetraminoanthraquinone (C. I. Disperse Blue 1), 1,4-diamino-5-nitroanthraquinone (C. I. Disperse Violet 8), etc. (the dye numbers are shown in parenthesis). Other than the above, examples of the usable dyes further include those that are known as C. I. Solvent Blue 14, C. I. Solvent Blue 35, C. I. Solvent Blue 63, C. I. Solvent Violet 13, C. I. Solvent Violet 14, C. I. Solvent Red 52, C. I. Solvent Red 114, C. I. Vat Blue 21, C. I. Vat Blue 30, C. I. Vat Violet 15, C. I. Vat Violet 17, C. I. Vat Red 19, C. I. Vat Red 28, C. I. Acid Blue 23, C. I. Acid Blue 80, C. I. Acid Violet 43, C. I. Acid Violet 48, C. I. Acid Red 81, C. I. Acid Red 83, C. I. Reactive Blue 4, C. I. Reactive Blue 19, C. I. Disperse Blue 7, and the like. Such anthraquinone dyes may be used singly or in a combination of two or more. Preferred among these anthraquinone dyes are C. I. Disperse Blue 7, C. I. Disperse Violet 1, etc. In the present invention, by modifying the type (molecular structure, etc.) of such anthraquinone dyes, detection sensitivity can be controlled.

Azo dyes usable herein are not limited so long as they contain, as a chromophore, an azo group (-N=N-). Examples include monoazo dyes, polyazo dyes, metal complex salt azo dyes, stilbene azo dyes, thiazole azo dyes, etc. Specific examples include, by dye number, C. I. Solvent Red 1, C. I. Solvent Red 3, C. I. Solvent Red 23, C. I. Disperse Red 13, C. I. Disperse Red 52, C. I. Disperse Violet 24, C. I. Disperse Blue 44, C. I. Disperse Red 58, C. I. Disperse Red 88, C. I. Disperse Yellow 23, C. I. Disperse Orange 1, C. I. Disperse Orange 5, and the like. These can be used singly or in a combination of two or more.

Usable methine dyes may be any of those as long as they contain a methine group. Therefore, in the present invention, the methine dyes include polymethine dyes, cyanine dyes, etc. These can be suitably selected from known or commercially available methine dyes. Specific examples include C. I. Basic Red 12, C. I. Basic Red 13, C. I. Basic Red 14, C. I. Basic Red 15, C. I. Basic Red 27, C. I. Basic Red 35, C. I. Basic Red 36, C. I. Basic Red 37, C. I. Basic Red 45, C. I. Basic Red 48, C. I. Basic Yellow 11, C. I. Basic Yellow 12, C. I. Basic Yellow 13, C. I. Basic Yellow 14, C. I. Basic Yellow 21, C. I. Basic Yellow 22, C. I. Basic Yellow 23, C. I. Basic Yellow 24, C. I. Basic Violet 7, C. I. Basic Violet 15, C. I. Basic Violet 16, C. I. Basic Violet 20, C. I. Basic Violet 21, C. I. Basic Violet 39, C. I. Basic Blue 62, C. I. Basic Blue 63, and the like. These can be used singly or in a combination of two or more.

The content of such a colorant contained in the ink composition for forming the second-color change layer can be suitably determined according to the type of the colorant, the desired hue, etc. However, it is preferable that the proportion of the colorant generally be from about 0.05 wt.% to about 5 wt.%, in particular 0.1 wt.% to 1 wt.%, relative to the ink composition.

In the present invention, the color-change layer (including the first-color change layer and second-color change layer; hereunder simply referred to as "color-change layer") and non-color change layer can be formed, using each of the inks, according to a known printing procedure, such as silk screen printing, gravure printing, offset printing, relief printing, flexographic printing, and the like. The printing order of the color-change layer and non-color change layer is not critical, and may be suitably determined depending on the design etc. to be printed.

The ozone indicator of the present invention may comprise one color-change layer and one non-color change layer; the indicator may also be formed of a plurality of laminated layers. Alternatively, at least one color-change layer, and at least one non-color change layer may be laminated together. In such a case, each of the color-change layers may have the same or different composition; likewise, each of the non-color change layers may have the same or different composition.

The color-change layer and non-color change layer may be formed all over or partially on the substrate, or each layer. In such cases, according to the present invention, the color-change layer and non-color layer are formed in such a manner that a part or all of at least one color-change layer can be exposed to the ozone atmosphere so as to ensure that the color-change layer undergoes a color change.

In the present invention, so long as the existence of ozone can be detected, the color-change layer and non-color change layer may be formed in such a manner that the color difference between the layers can be recognized after the color-change layer undergoes a color change; alternatively, the color-change layer and non-color change layer may be formed in such a manner that the layers become the same color after the color-change layer undergoes a color change. In particular, it is preferable to form the color-change layer and non-color change layer in such a manner that the color difference between the layers can be recognized after the color-change layer undergoes a color change.

For recognition of the color difference between the color-change layer and non-color change layer, the layers may be, for example, formed in such a manner that at least one selected from a letter, a pattern, and a symbol appears after the color-change layer undergoes a color change. In the present invention, any letters, patterns and symbols may be employed as long as they can be used for recognition of a color change (i.e., the existence of ozone). Such letters, etc., may be suitably designed according to the purpose of its usage, etc.

The color-change layer and non-color change layer may be of different colors before a color change is observed. It is also possible to form the layers substantially of the same color so that the color difference (contrast) can be observed between the color-change layer and non-color layer, after a color change.

The ozone indicator of the present invention may be formed in such a manner that the color-change layer and non-color change layer are not superimposed. With such an arrangement, the amount of ink used can be lessened.

In the present invention, either a color-change layer or non-color change layer may further be applied on at least one layer selected from the color-change layer and non-color change layer. For example, the color-change layer and non-color layer may be formed so as not to superimpose each other (hereunder referred to as "color-change/non-color change layer") and a color-change layer having a different design further laminated thereon; such a formation makes the boundary line between the color-change layer and non-color layer in the color-change/non-color change layer substantially indiscernible. Thereby, the more excellent appearance of the ozone indicator can be achieved.

The ozone indicator of the present invention undergoes a color change in the high concentration range with a CT value (ozone concentration x exposure time) of generally 300 ppm-min or more, particularly 500 ppm-min or more, preferably 1000 ppm-min or more, and still more preferably 3000 ppm-min or more. The upper CT value limit is not limited, but is generally about 100000 ppm-min. The same effect can be obtained even when the color-change layer of the ozone indicator is formed so as to be exposed directly to the ozone atmosphere. In other words, according to the ozone indicator of the present invention, a color-change layer may be formed without having a protective layer etc. thereon.

Regarding water vapor, the indicator of the present invention can detect ozone with accuracy under humidity in the range of generally 10% or more, particularly under high humidity of 90% or more.

Therefore, the ozone indicator of the present invention can be preferably used in, for example, an apparatus for sterilizing an object using ozone water, as an ozone indicator for detecting ozone gas generated from the ozone water. Such ozone gas is highly concentrated, and contains a large amount of water vapor. It is difficult for a conventional ozone indicator to detect ozone with accuracy in such a high-concentration and high-humidity ozone atmosphere. The ozone indicator of the present invention is, however, capable of detecting an ozone exposure time or a CT value (CT value = concentration x exposure time) with higher accuracy even in such an atmosphere.

### EXAMPLES

The present invention is described in further detail with reference to the following Examples. However, the present invention is not limited to the Examples. Examples and Comparative Examples

The components listed in Table 1 were uniformly mixed using an agitator to prepare ink compositions. Specifically, the solvent and dye were first mixed and stirred using a dissolver. Subsequently, the resinous binder and non-color change pigment were added thereto, and further stirred. The temperature of the mixture was then returned to room temperature, and the surfactant and extender were added thereto. The resulting mixture was uniformly stirred to obtain an ink composition. In Table 1, the Examples are from No. 1 to No. 2, and the Comparative Examples are from No. 3 to No. 7.

**Table 1**

| Composition | Example | | Comparative Example | | | | | |
|---|---|---|---|---|---|---|---|---|
| | 1 | 2 | 3 | 4 | 5 | 6 | 7 | |
| C. I. Basic Blue 3 | 1.60 | 1.60 | | | | | | |
| C. I. Basic Red 13 | | | 1.60 | | 1.60 | | | |
| C. I. Disperse Violet 1 | | | | | | | 1.60 | |
| C. I. Basic Violet 7 | | | | 1.60 | | 1.60 | | |
| Butyl cellosolve | 79.40 | 79.40 | 79.40 | 79.40 | 79.40 | 79.40 | 79.40 | |
| Microlith Yellow 3R-T | 1.00 | 1.00 | 1.00 | 1.00 | 1.00 | 1.00 | 1.00 | |
| Joncryl 690 | 3.50 | 3.50 | 3.50 | 3.50 | 3.50 | 3.50 | 3.50 | |
| Nitrocellulose HIG1/2 | | | | | 6.50 | 6.50 | 6.50 | |
| Ethocel 10 | 6.50 | 6.50 | 6.50 | 6.50 | | | | |
| NIKKOL CA-2580 | 1.00 | | 1.00 | 1.00 | 1.00 | 1.00 | 1.00 | |
| NIKKOL CA-2150 | | 1.00 | | | | | | |
| Aerosil R-972 | 7.00 | 7.00 | 7.00 | 7.00 | 7.00 | 7.00 | 7.00 | |
| Total | 100.00 | 100.00 | 100.00 | 100.00 | 100.00 | 100.00 | 100.00 | |
| Colon Change | Green to Yellow | Green to Yellow | Red-purple to Yellow | Red-purple to Yellow | Red-purple to Yellow | Purple to Yellow | Purple to Yellow | |
| *Test 1 | ○ | ○ | X | X | X | X | X | |
| | | | | | | | | |

| | | | | | | | | |
|---|---|---|---|---|---|---|---|---|
| *Test 1 ... Ozone Exposure Test with CT value of 5,000 ppm-min under ambient temperature and humidity of 80% or more. When the color change degree of ΔE is 15 or more, "○" is shown; when ΔE is less than 15, "X" is shown. | | | | | | | | |

The component shown in Table 1 is specifically explained as follows.
(1) C. I. Basic Blue 3: oxazine dyes
(2) C. I. Basic Red 13: methine dyes
(3) C. I. Disperse Violet 7: azo dyes
(4) C. I. Basic Violet 7: methine dyes
(5) Butyl cellosolve: solvent
(6) Microlith Yellow 3R-T: a non-color change pigment; Product Name: "Microlith Yellow 3R-T", produced by Chiba Specialty Chemicals Co., Ltd.
(7) Joncryl 690: a resinous binder; Product Name: "Joncryl 690", produced by Johnson Polymer Ltd.
(8) Nitrocellulose HIG 1/2: a resinous binder; Product Name: "Nitrocellulose HIG 1/2", produced by Daicel FineChem Ltd.
(9) Ethocel 10: a resinous binder; Product Name: "Ethocel 10", produced by Dow Chemical Co., Ltd.
(10) NIKKOL CA-2580: a quaternary ammonium salt-type surfactant; Product Name: "NIKKOL CA-2580", produced by Nikko Chemicals Co. Ltd.
(11) NIKKOL CA-2150: a quaternary ammonium salt-type surfactant; Product Name: "NIKKOL CA-2150", produced by Nikko Chemicals Co. Ltd.
(12) Aerosil R-972: a silica gel; Product Name: "Aerosil R-972", produced by Aerosil Japan Ltd.

### TEST EXAMPLE 1

The ink compositions obtained in the Examples and Comparative Examples were examined with respect to the color change and the degree thereof. Using each of the ink compositions, a coating film was formed on a high-quality paper (50cm x 50mm) by 250 mesh-screen printing. The resulting coating films were sufficiently dried to use as samples.

The color change properties of the samples were evaluated by allowing them to be exposed to ozone at an ambient temperature, and from ambient humidity to humidity of 80%, with a CT value of 5000 ppm-min (ozone level: 1000 ppm). When the color change degree of ΔE was 15 or more, the sample was evaluated as "O"; when the degree of ΔE was less than 15, the sample was evaluated as "'X". The results are also shown in Table 1.

## Claims

1. An ink composition for ozone detection comprising an oxazine dye and a cationic surfactant, and not comprising colorants (other than the oxazine dyes) that undergo a color change upon exposure to ozone.

2. The ink composition for ozone detection according to Claim 1, wherein the cationic surfactant is a quaternary ammonium salt-type cationic surfactant.

3. The ink composition for ozone detection according to Claim 2, wherein the cationic surfactant is an alkyl trimethylammonium salt.

4. The ink composition for ozone detection according to Claim 1, further comprising an extender.

5. The ink composition for ozone detection according to Claim 1, further comprising a resinous binder.

6. An ozone indicator comprising a first-color change layer formed from the ink composition for ozone detection according to Claim 1.

7. The ozone indicator according to Claim 6, comprising, on a substrate, (1) at least one layer selected from (a) a second-color change layer (not including oxazine dyes) that undergoes a color change upon exposure to ozone, and (b) a non-color change layer that does not undergo a color change upon exposure to ozone; and (2) the first-color change layer, wherein each of the layers are laminated in such a manner that a part or all of each color-change layer can be exposed to an ozone atmosphere.

8. The ozone indicator according to Claim 7, having at least one second-color change layer.

9. The ozone indicator according to Claim 8, wherein the first-color change layer and second-color change layer undergo a color change upon different ozone concentrations and/or ozone exposure times.

10. The ozone indicator according to Claim 6 for use in detecting ozone in an ozone-gas atmosphere with a CT value of 1000 ppm-min or more.

11. The ozone indicator according to Claim 10, wherein atmospheric humidity is 90% or more.
